# EUROPEAN PATENT SPECIFICATION

(11) **EP 3 954 398 B1**
(45) Date of publication and mention of the grant of the patent: **12.07.2023**
(21) Application number: 21150626.6
(22) Date of filing: 07.01.2021
(51) Int. Cl.: A61L 2/18, A61L 2/22, A61L 9/14, A61L 2/03, F24F 3/16, F24F 6/14, F24F 8/133, F24F 8/15, F24F 11/30, F24F 110/10, F24F 110/20

(54) **METHOD AND SYSTEM FOR DISINFECTING AIR**
VERFAHREN UND SYSTEM ZUR DESINFEKTION VON LUFT
PROCÉDÉ ET SYSTÈME DE DÉSINFECTION DE L'AIR

(30) Priority: 10.08.2020 EP 20190355; 03.09.2020 EP 20194456
(43) Date of publication of application: 16.02.2022
(73) Proprietor: oji Europe GmbH, 14641 Nauen (DE)
(72) Inventor: Bone-Winkel, Thomas, 14641 Nauen (DE); Singh, Gundeep, 14641 Nauen (DE); Boecker, Dirk, 14641 Nauen (DE)
(74) Representative: RGTH

(56) References cited:
- EP-A1- 3 150 934
- EP-B1- 3 150 934
- WO-A1-2019/193828
- CN-A- 111 481 723
- JP-A- 2004 141 429
- JP-A- 2006 305 321
- JP-A- 2018 050 483

## Description

The present invention relates to a method for disinfecting air, wherein a disinfectant is dispersed into a volume to be disinfected, wherein the disinfectant comprises electrolyzed and/or sterilized water containing hypochlorous acid. The present invention further relates to a system for disinfecting air.

### Technological background

Hypochlorous acid (HOCI) is a weak acid that forms when chlorine dissolves in water, and itself partially dissociates, forming hypochlorite ClO⁻. The ratios of HOCI, ClO⁻ and Cl₂ in the resulting aqueous solution are pH dependent. Hypochlorous acid (HOCI) and ClO⁻ are oxidizers, and the primary disinfection agents of chlorine solutions.

HOCI is an endogenous substance in all mammals and is effective against a broad range of microorganisms. Neutrophils, eosinophils, mononuclear phagocytes and B-lymphocytes produce HOCI in response to injury and infection through the mitochondrial membrane-bound enzyme known as "respiratory burst nicotinamide adenine dinucleotide phosphate oxidase". In aqueous solution, HOCI dissociates into H⁺ and ClO⁻, denaturing and aggregating proteins. HOCI also destroys viruses through chlorination by forming chloramines and nitrogen centered radicals, which can result in single- as well as a double-stranded DNA breaks for non-enveloped sensors, rendering the nuclear acid useless and the virus harmless. An enveloped virus such as a Corona virus, will be deactivated by the chlorine components (HOCI, ClO⁻, and Cl₂) through reacting with and incapacitating their lipid shell/envelope. An intact lipid shell is mandatory for the virus to link to its target cells and ultimately reproduce.

HOCI has been used, for example, in eye care, where it can provide effective relieve from dry eyes and hordeola. HOCI has also been used for the treatment of wounds, for water treatment in water supply systems or to neutralize odors.

Microorganisms, in particular viruses and bacteria, are present everywhere in everyday life. Some of these viruses and microorganisms can be harmful to mammals in general and to humans in particular. With the COVID-19 epidemic caused by the SARS-CoV-2 virus the need for methods and systems for the disinfection of air and surfaces has grown even more.

JP 2006-305321 A discloses an air purification device that uses an electrostatic atomization technique to capture and remove malodors, airborne bacteria, molds, allergen substances, etc. floating in a room in the air.

WO 2019/193828 A1 discloses a sterilizing water sprayer capable of spraying antibacterial sterilizing water and effectively suppressing the growth of microorganisms such as mold in the bathroom.

JP H11-169441 A discloses an indoor sterilization method for non-populated rooms capable of simultaneously sterilizing not only ceilings and walls but also the air by atomization of a disinfectant. The disinfectant is sterilized water containing hypochlorite with a pH between 3 and 7.5 and having a hypochlorous acid concentration of 10 to 200 ppm.

JP 2007-162997 A discloses a dry fog generator suitable for a waiting room or a patient's room of a hospital, for sterilizing or sanitizing floating fungus by nebulizing and emitting a chemical. The chemical is a hypochlorite solution, which is sprayed from a nozzle of the dry fog generator.

Prior art document US 2014/0119992 A1 discloses a disinfecting solution comprising a liquid having an HOCI concentration between 50 ppm and 4.000 ppm and a quantity of ethyl alcohol comprising between 0.05% and about 10% of the disinfectant solution by weight.

WO 2011/085470 A1 provides a delivery mechanism for dispersing a pathogen disinfectant liquid into the air or onto surfaces. The apparatus includes a container comprising a sunlight-resistant compartment containing a pathogen disinfectant liquid, a propellant, and an actuator coupled to a valve for opening and closing the valve, wherein opening of the valve causes the propellant to move at least a portion of the pathogen disinfectant liquid through an orifice in the container.

Prior art document CN 111481723 A discloses a method for disinfecting air in gathering places by using a weak hypochlorous acid disinfectant wherein the weak hypochlorous acid disinfectant has a pH of 5.0 to 6.5 and contains 10 ppm to 500 ppm hypochlorous acid and 10 ppm to 9.000 ppm sodium chloride. A cold fogging machine using high frequency ultrasonic vibrations between 5 kHz to 1.7 MHz disperses the weak hypochlorous acid disinfectant into the air at a rate of 0.5 to 1.5 l/hour.

While the dispersion of disinfectants containing HOCI into the air with dispersion rates between 0.5 to 1.5 l/hour is useful for disinfection of surfaces, these high dispersion rates have the disadvantage, that the disinfectant is emitted as a dense fog. The emission of such dense fogs is not only visually unpleasing, but it also prevents a continuous dispersion of the disinfectant over long periods of time, when animals or humans are present. At dispersion rates of 0.5 to 1.5 l/hour the humidity air is increased to almost 100 % and the disinfectant can be damaging to equipment and surfaces present in the volume or area. Therefore, in the prior art the disinfectants are emitted in short bursts, the bursts being distributed over the course of the day. The disinfection results from the known methods are spotty and often arbitrary. For example, some surfaces such as table tops, in the same room are disinfected while other surfaces are not disinfected at all, due to the dispersion of the disinfectants in a practically uncontrolled manner. In the prior art a disinfection of venues or locations with people present is unfeasible because of the negative effect of the disinfectant when dispersed at the proposed high rates.

### Disclosure of the invention: problem, solution, advantages

It is an object of the present invention to provide a method for disinfecting air which provides a continuous protection of people and/or animals from pathogens in a given volume, in particular for essentially 24 hours a day, and which can be conducted with people and/or animals present. The pathogens from which the people and/or animals can be protected could be spread homogeneously throughout the given volume.

To solve the object the invention proposes a method for disinfecting air, wherein a disinfectant is dispersed into a volume to be disinfected, wherein the disinfectant comprises electrolyzed and/or sterilized water containing hypochlorous acid, wherein a dispersion rate of the disinfectant is adjusted such, that a concentration of the hypochlorous acid in the volume is continuously between 0.05 mg/m³ and 2.0 mg/m³.

The unit m³ in the concentration relates to the volume to be disinfected.

The disinfectant can be dispersed continuously in time or the disinfectant can be dispersed periodically or interval based or aperiodically. Preferably, the dispersion rate of the disinfectant can be variable.

In the inventive method the disinfectant is dispersed at a, preferably variable, dispersion rate into the volume so that a concentration of hypochlorous acid in the volume lies continuously between 0.05 mg/m³ and 2.0 mg/m³. In effect, a grid of the disinfectant, in particular of "breathable HOCI", is created within the volume for protection of people and/or animals from pathogens with a concentration of the disinfectant which is, within the proposed range, homogeneous in time and space. The term "breathable HOCI" may imply that it can be inhaled over long periods of time without any harm to the body of humans or animals or its functions. Because of the proposed concentration of the hypochlorous acid, people and/or animals may be present in the volume while the method is conducted without any negative effects on their wellbeing.

Preferably, the concentration of hypochlorous acid can be adjusted while the method is being conducted. Thus, if boundary conditions in the volume change over time, for example if the number of people and/or animals present changes or if the temperature changes, the concentration may be adjusted in order to maintain a continuous and effective disinfection of the air within the volume. The adjustment of the concentration can be automatic or manually driven.

According to the invention, the concentration of the hypochlorous acid in the volume lies continuously between 0.05 mg/m³ and 2.0 mg/m³. In particular when humans are present in the given volume an upper bound of 2.0 mg/m³ for the concentration of the hypochlorous acid in the volume may be chosen, so that the concentration of the hypochlorous acid in the volume is continuously between 0.05 mg/m³ and 2.0 mg/m³.

A higher upper bound of 10.0 mg/m³ for the concentration of the hypochlorous acid in the volume is preferable when only animals are present in the given volume.

Preferably, the concentration may be adjusted to be between 0.1 mg/m³ and 2.0 mg/m³, further preferably between 0.1 mg/m³ and 1.0 mg/m³, still further preferably between 0.2 mg/m³ and 0.5 mg/m³. In addition, the range of the concentration may change over time. For example, at a first point in time t₁ the concentration may lie between 0.3 mg/m³ and 1.0 mg/m³, and at a later point in time t₂ the concentration may lie between 0.05 mg/m³ and 0.5 mg/m³. The range may be adjusted in particular in response to changes of the conditions within the volume.

If, preferably only, animals are present in the given volume the concentration may be adjusted to higher levels. For example, the concentration may be adjusted to be between 1.0 mg/m³ and 8.0 mg/m³, preferably between 3.0 mg/m³ and 7.0 mg/m³.

It may still further be preferred, that at the start of the method a high dispersion rate is selected in order to reach the proposed range of the concentration of hypochlorous acid within a short period of time. Afterwards, the dispersion rate may be lowered to a level that ensures that the concentration of hypochlorous acid remains within the proposed range.

The dispersion rate of the disinfectant may be between 0.01 ml/h and 100 ml/h, preferably between 0.1 ml/h and 25 ml/h, further preferably between 0.5 ml/ h and 12 ml/h, depending on the volume to be disinfected, and/or on the concentration of hypochlorous acid in the volume and/or in the disinfectant, and/or on the number of dispersion apparatus required to reach and maintain the proposed concentration of the hypochlorous acid in the volume. A suitable dispersion apparatus is described below. A single dispersion apparatus or multiple dispersion apparatus may be used for dispersing the disinfectant.

It is still further preferred that the concentration and/or the dispersion rate is adjusted based on at least one of the parameters: volume to be disinfected, concentration of hypochlorous acid in the volume and/or in the disinfectant, and/or air exchange rate, and/or ventilation status, and/or humidity, and/or atmospheric pressure, and/or surface area and/or material of surfaces present in the volume, and/or characteristics of walls surrounding the volume, for example material and surface structure, and/or a decay rate of disinfectant, and/or number of people present in the volume, and/or temperature and/or pH of the air in the volume, and/or concentration of gases in the volume, for example the concentration of CO₂, Cl₂, ClO₂, HOCI, N₂, H₂CO, H₂O, CIO, HCl, and/or at least one of PM 0.3, PM 1.0, PM 2.5, PM 10, HCHO concentration, TVOC concentration.

The PM value describes the amount of particulate matter in the air in units µg/m³. For example, PM 1.0 particles relates to the concentration of particulate matter with a diameter of less than 1.0 µm.

The amount of effective HOCI in the air can be determined by using a complex method of measuring components or gases like CLO⁻, Cl₂, HCL and NO₂ to arrive at a number for referencing the amount of HOCI in the air at any point in time.

By using a displacement method displaced gases may be calculated to verify the net concentration of HOCI at any point in time in the volume.

By measuring the concentration of Cl₂ and ClO· radicals in the air the net concentration of HOCI can be determined to calibrate the dispersion rate. The concentration of HOCI in the volume can be measured via the HOCI dissociation components.

By measuring the PM 0.3, PM 1.0, PM 2.5, PM 10 levels particles the size of bacteria and viruses in the air can be detected and the concentration and/or the dispersion rate can be adjusted accordingly.

Furthermore, to ensure that surfaces and air are disinfected and to measure the net effectiveness of the method, a surrogate process using ATP (adenosine triphosphate) measurement kits to measure and understand residual CFUs (colony forming unit) on surfaces can be used. In this way the net efficacy on surfaces of the dispersion can be recorded.

The ATP measurement is an indicator of actively growing microorganisms by measuring adenosine triphosphate using light. The CFU is a measure to estimate the number of viable bacteria or fungal cells in a sample.

The air exchange rate can be the exchange rate of the air inside the volume to be disinfected, the humidity can be the room humidity. By, preferably dynamically, adjusting the dispersion rate the concentration of hypochlorous acid in the volume to be disinfected can be controlled within the optimum ranges to neutralize pathogens during the dispersion process.

The, preferably time dependent, concentration and/or dispersion rate may be controlled and/or adjusted by a control device executing a software algorithm. The software algorithm may be provided with at least a subset of the parameters described above and may, based on the parameters, determine the required concentration of hypochlorous acid and/or the dispersion rate over time. The algorithm may determine the concentration of hypochlorous acid and/or the dispersion rate prior to the dispersion of the disinfectant or continuously and/or periodically during dispersion of the disinfectant.

For example, prior to the dispersion of the disinfectant, the algorithm may be provided with at least some of the parameters described above, for example the volume to be disinfected, the air temperature in the volume, surface materials present in the volume and the number of people expected to be present in the volume. The algorithm then determines the required concentration of hypochlorous acid, which may vary over time, as well as the required dispersion rate, which may also vary over time, to reach and maintain the determined concentration of hypochlorous acid. The algorithm may also determine whether the disinfectant is dispersed continuously in time, periodically or interval based or aperiodically, or any combination thereof.

It may also be preferred that the parameters are continuously or periodically updated. The software algorithm may receive the updated parameters continuously or periodically and, based on the updated parameters, may continuously or periodically, preferably in real-time, adjust the concentration and/or the dispersion rate.

For updating the parameters, the control device may receive data from sensors distributed within the volume and feed the data to the software algorithm. The sensors may be sensors for measuring the volume to be disinfected, a concentration of hypochlorous acid in the volume or in the disinfectant, and/or an air exchange rate, and/or a ventilation status, and/or humidity, and/or atmospheric pressure, and/or surface area and/or material of surfaces present in the volume, characteristics of walls surrounding the volume, for example material and surface structure, and/or a decay rate of disinfectant, and/or number of people present in the volume, and/or temperature and/or pH of the air in the volume, and/or concentration of gases in the volume, for example the concentration of CO₂, Cl₂, ClO₂, HOCl, N₂, H₂CO, H₂O, ClO⁻, HCl, and/or at least one of PM 0.3, PM 1.0, PM 2.5, PM 10, HCHO concentration, TVOC concentration.

The software algorithms may preferably be configured to calculate exact deposition and absorption rates of the disinfectant and/or of hypochlorous acid on all surfaces and structures within the volume.

The control device may have a range of connectivity options available to monitor and control the device. The device may provide wireless connectivity through BLE, WiFi, 2G, 4G, 5G or NB-loT. The control device may send and/or receive data, in particular from the sensors via at least one of BLE, 2G, 4G, 5G, Wi-Fi, LAN or NB-loT. In addition, the control device may be able to send or receive data from and to the internet. Part or all of the determination of the concentration and/or the dispersion rate may be conducted by an external server, for example by a cloud computer. The determination of the concentration and/or the dispersion rate may be provided via a SaaS platform. The control device and/or the conduction of the method may be accessed, monitored and controlled using mobile applications.

The method may be controlled remotely. A warning system may be implemented which provides a virus load warning due to high HOCI consumption, a ventilation control warning, etc. Via the SaaS platform and/or the mobile application all parameters may be controlled for an automated and controlled dispersion of the disinfectant.

The sensors may be part of a sensor kit. The sensor kit may be configured to measure any combination of the parameters described above.

Still further preferably the disinfectant is dispersed as an aerosol, wherein a mean value of a droplet size distribution is between 0.5 µm and 50 µm, preferably between 1 µm and 10 µm, further preferably between 2 µm and 8 µm, particularly preferably between 3 µm and 7 µm.

By dispersing the disinfectant as an aerosol comprising droplets with sizes of about 0.5 to 50 µm, the disinfectant droplets can stay afloat. The lower the droplet size, the longer the droplets stay afloat up to perpetuity.

With droplet sizes between 3-10 µm or below, the disinfectant can stay afloat virtually indefinitely. When the droplet size is below 3-10 µm, the droplets evaporate rapidly resulting in a disinfectant and/or hypochlorous acid in the form of sub-micron size particles, which never settle down because of gravity. The speed of the evaporation depends on the droplet size, the room temperature and humidity. Accordingly, the evaporation may be controlled by adjusting the droplet size, the room temperature and humidity.

The concentration of the, preferably sub-micron size, particles of the aerosol of the disinfectant and/or of the hypochlorous acid is reduced by the disinfectant particles hitting surfaces within the volume, thereby disinfecting said surfaces. Other factors for the reduction of the concentration are virus and bacterial load in the volume, surfaces, air exchange rate, for example due to ventilation or the use of HVAC systems, or the breathing rate of people or animals present in the volume. Thus, the dispersion rate may be adjusted to compensate for these effects.

The disinfectant is preferably dispersed as an aerosol and shortly thereafter distributes in the volume and evaporates, preferably into sub-micron size particles.

Accordingly, it is preferred that the disinfectant and/or hypochlorous acid in the volume is present in the form of sub-micron size particles.

The sub-micron size particles of the disinfectant and/or of the hypochlorous acid float in the air searching for organic matter to interact and deactivate a large portion of unwanted bacteria and viruses in the air. At the same time, any contamination on the surfaces is also deactivated. While it is working on the pathogens, HOCI also neutralizes any unwanted odors in the air.

With larger droplet sizes, for example between 20µm and 50µm the droplets may stay afloat for a limited amount of time, e.g. up to 1 hour or longer, in the air inside the volume. Thus, even with larger droplet sizes the disinfectant can diffuse homogeneously throughout the volume to be disinfected. Because of gravity the droplets having larger droplet sizes will continuously and slowly sink down and eventually settle on the surfaces present in the volume or area, providing an additional disinfection and/or coating of surfaces for up to 96 hours.

The disinfectant comprising electrolyzed and/or sterilized water containing hypochlorous acid is not harmful to humans or animals. Therefore, when present with a hypochlorous acid concentration between 0.05 mg/m³ and 2.0 mg/m³, the air inside the volume can be inhaled without disturbing humans or animals and without causing any harm on them. To the contrary, the disinfectant comprising electrolyzed and/or sterilized water containing hypochlorous acid has beneficial health effects due to its disinfecting ability and therefore pathogen reducing effects.

The method of the present invention is the first disinfection method for air which provides a truly continuous protection homogeneously distributed in spaces or volumes where people and/or animals are present.

The disinfectant, in particular the electrolyzed and/or sterilized water, can be positively charged electrolyzed water (EW+) or negatively charged electrolyzed water (EW-). However, the electrolyzed and/or sterilized water may also be not charged.

The method can be carried out in areas with or without humans or animals present. The method may be carried out in gathering places, office buildings, schools, universities, private homes, and open areas such as parks or sporting stadiums. The method may furthermore be carried out in any public, retail or industrial locations, in railway stations, concert halls, malls, administrative buildings, hotels, hospitals, retirement homes, and in the meat, milk, or egg production.

The method maybe furthermore carried out in the veterinary field or in the field of animal husbandry, livestock farming or animal breeding. Advantageously, when carried out in these fields the amount of antibiotics and antiviral substances can significantly be reduced compared to the prior art.

By selecting an expedient concentration of hypochlorous acid within the proposed range, the method can be carried out in various areas and fields of applications. For example, when the method is carried out in closed gathering places a lower concentration between 0.1 mg/m³ and 0.3 mg/m³ may be chosen compared to open gathering spaces. When the method is carried out in the field of animal husbandry a higher concentration may be suitable, for example a concentration between 0.3 mg/m³ and 3.0 mg/m³ or even higher may be chosen.

Preferably, the disinfectant is dispersed at a dispersion rate such that the concentration of hypochlorous acid remains continuously within the proposed range for at least 12 hours a day, further preferably for at least 16 hours a day, still further preferably for at least 20 hours a day, particularly preferably for essentially 24 hours a day. In particular, the concentration of hypochlorous acid may remain within the proposed range for 24 hours a day.

Preferably the volume to be disinfected is between 3 m³ and 150.000 m³, further preferably between 100 m³ and 50.000 m³, still further preferably between 1.000 m³ and 10.000 m³, particularly preferably between 2.000 m³ and 5.000 m³. Furthermore preferably, the volume to be disinfected is between 50 m³ and 200 m³, which is the typical volume of commercial or office spaces.

Thus, the method is suitable for disinfection of a large range of volumes and/or areas.

The disinfectant can be dispersed into very small volumes, for example small rooms, and into very large volumes, for example stadiums, convention centers, large office buildings and the like.

Further preferably, the disinfectant has a pH between 4.5 and 9, preferably between 6 and 8, further preferably between 6.5 and 7.5.

It is also possible that the disinfectant has a pH between 4 and 7, preferably between 5 and 6. With a pH value between 5 and 6 the HOCI content in the dispersed disinfectant can be increased compared to a higher pH value.

Thus, the disinfectant of the method is preferably neutral or only slightly acidic or slightly basic. This, inter alia, allows for the disinfectant to be inhaled by humans or animals without harm.

Preferably, the disinfectant does comprise a small, preferably minimal, number of sodium-ions, sodium chloride or dissolved sodium hypo chlorite (NaOCI) or sodium chlorite (NaClO₂).

Further preferably, the concentration of sodium-ions, and/or sodium chloride and/or dissolved sodium hypo chlorite and/or sodium chlorite in the disinfectant is below 100 ppm, further preferably below 50 ppm, still further preferably below 10 ppm, still further preferably below 7 ppm.

The disinfectant may be created by electrolysis of NaCl solutions or electrolysis of hydrochloric acid solution or electrolysis of sodium chloride with cavitation. The oxidation of the Cl⁻ Ions at the anode yields Cl₂ gas which may be guided to a water tank where it reacts to HOCI and HCl.

Since the disinfectant is thus not salty, the risk of corrosion of surfaces in the volume and/or area is greatly reduced.

Preferably, the hypochlorous acid has a concentration in the disinfectant between 10 ppm and 1.000 ppm, further preferably between 100 ppm and 500 ppm, still further preferably between 200 ppm and 400 ppm. The hypochlorous acid may also have a concentration in the disinfectant between 400 ppm and 1.000 ppm, preferably between 500 ppm and 800 ppm.

When a highly concentrated HOCI solution, for example with 500 ppm to 800 ppm of HOCI is used, the fluid volume and the build-up time to reach the proposed concentration of the airborne hypochlorous acid can be reduced. Also, smaller disinfectant reservoirs can be used.

Further preferably the disinfectant comprises at least one additive.

By adding at least one additive, further beneficial effects can be obtained. For example, the additives may increase the disinfection capability of the disinfectant used in the method. An additive may increase the formation of HOCI containing sub-micron size particles when the disinfectant is released from an aerosol creating machine. Also, the additives may be odors, which improve the room quality and are pleasant to persons present. Furthermore, the additives may be stabilizers that stabilize the disinfectant, in particular the hypochlorous acid in the disinfectant.

The additives may also be suited to enhance breathing of people and/or animals, such as menthol.

There may be one or more additives present, which can be chosen independently from each other.

Further preferably the additive is present in the disinfectant with a weight percentage or a volume percentage between 0.01 % and 5.0 %, preferably between 0.25 % and 1.0 %, further preferably between 0.5 % and 0.75 %.

If more than one additive is present, each of the additives may be present in the disinfectant with a weight percentage or a volume percentage between 0.01 % and 5.0 %, preferably between 0.25 % and 1.0 %, further preferably between 0.5 % and 0.75 %.

Still further preferably the at least one additive comprises an essential oil, wherein the essential oil further preferably is at least one out of cinnamon oil, carvacrol oil, thymol oil, cajuput oil, clove oil, eucalyptus oil, sage oil, tea tree oil.

The use of essential oils is particularly beneficial since essential oils may have themselves a disinfecting effect, for example thymol oil. In addition, essential oils provide a nice and pleasant odor. For different open or closed spaces or volumes and/or for different times of the day and/or for different applications, different additives, in particular different essential oils, can be selected.

While it is preferred, that the essential oils are contained in the disinfectant, it is also possible, that the essential oils are dispersed separately, for example with a separate fogging generator, via a heating, ventilation, and air conditioning system (HVAC) or by any other suitable means.

Furthermore, the additives may comprise a mixture of different essential oils.

Still further preferably the disinfectant has a redox potential between 500 mV and 1.500 mV, preferably between 700 mV and 1.200 mV, further preferably between 900 mV and 1.000 mV.

Because of the advantageous level of the redox potential the disinfectant can be inhaled without any harmful impact on humans or animals. Furthermore, the disinfectant does not have disadvantageous effects on surfaces such as corrosion or the like.

It is still further preferred that the disinfectant is dispersed via a heating, ventilation, and air conditioning system (HVAC). The use of an HVAC system to disperse and distribute the disinfectant allows for the disinfectant to be dispersed in a large building or throughout multiple rooms at the same time.

Preferably, the air of the volume is filtered and/or sanitized. By filtration and/or sanitization of the air of the volume, impurities in the air can be reduced. Particularly preferably, the filtration and/or sanitization can be a low MERV11-16 or a H10-13 filtration. Further preferably, an activated carbon filter with or without an iodine filter, or a cold catalyst filter can be used. Still further preferably, a positive ions or plasma generator, MERV 12-18 and/or HEPA H13 to U17 filters may be used. It is also possible that the operation of an UV lamp or an ozone generator is combined with the dispersion of the disinfectant.

The filtration may be conducted in several, preferably subsequent, steps. For example, in one step the air may be filtered and/or sanitized using silver ion nanoparticles. In a further step, the air may be filtered and/or sanitized using a UV light activated TiO₂ coated grid that is photocatalytic and produces oxygen due to the activation by UV light. The combination of these steps provides high ORP potential for sanitizing any unwanted pathogens in the air. In a still further step, two layers of honeycomb carbon (110mm) (activated/catalytic) may be used. The two layers of honeycomb carbon remove bacteria, Odors, VOCs and HCHO from the air up to F4 level.

It may also be preferred that in a next step a negative ion generator is employed to produce negative ions for making the air quality better. Afterwards a multi-layered cold plasma reactor with preferably four cold plasma tubes may be used to generate positive and negative ions as well as low volume ozone to ensure that the air around is protected.

In a next step a HEPA 110 mm E13 or H13 medical grade filter may be used to ensure all particles are filtered and no residue is in the air to give an average PM 2.5 count of less than 30 as well as a reduction in PM 1.0.

The filtration of the air of the volume and/or area can be carried out by an HVAC system or with an independent filtration and/or sanitation device.

It is also possible, that the dispersed disinfectant, in particular the aerosol of the disinfectant, is not filtered, but that the dispersion of the disinfectant and the filtration of the air are carried out independently and in parallel.

However, it may be preferred that the dispersed disinfectant passes through the filters of the filtration and/or sanitation device or through the filters of the HVAC system. Studies have shown that viruses and bacteria can accumulate on filter materials. Because of this accumulation, cleaning personal cleaning or changing the filters of HVAC systems have been shown to have a higher infection risk from such filters. This problem is particularly prevalent in connection with HVAC and air purification systems in airplanes. It is therefore particularly advantageous that the dispersed disinfectant passes through the filters, for example of the HVAC systems or the filtration and/or sanitation device, so that the contamination of the filters with pathogens is reduced.

By combining the continuous dispersion of a disinfectant into a volume to be disinfected with the filtering and/or sanitation of the air of the volume, a multi-layered disinfection and air purification method is provided, which improves the process of reducing pathogens in the air in large and small areas.

The filtration and/or sanitation device may have wireless connectivity. In particular the filtration and/or sanitation device may send and/or receive data, in particular from the sensors or the control device via BLE, 2G, 4G, 5G, Wi-Fi, LAN or NB-IOT. In addition, the filtration and/or sanitation device may be able to send and/or receive data from and to the internet. The filtration and/or sanitation may be connected to an external server, for example to a cloud computer. The external server may be a SaaS platform. The filtration and/or sanitation device be accessed, monitored and controlled using mobile applications.

Preferably a concentration of at least one type of virus and/or bacteria in the volume and/or area, preferably on a surface in the volume and/or area, is reduced by a log reduction value between 2-log and 8-log, preferably between 3-log and 7-log, further preferably between 4-log and 6-log, still further preferably between 4-log and 5-log.

Thus, with the method between 99 % to 99.999999 % of bacterial and/or viruses can be neutralized. Particularly preferably between 99.99 % and 99.999 % of bacteria or viruses can be neutralized.

At least one of the following microorganisms can be neutralized with the method:
Aspergillus Niger, Aureobasidium pullulans, Acinetobacter Baumannii, Bacteroides Fragilis, Corynebacterium Diphtheriae, Clostridium Difficile (spores), Candida Albicans, Corynebacterium Amycolatum, Enterbacter Aerogenes, Escherichia Coli, Enterococcus Faecalis - VRE MDR, Hemophilus Influenzae, Klebsiella Pneumoniae, Klebsiella Oxytoca, Malassezia Furfur, Micrococcus Yunnanesis, Methicillin- Resistant Staphylococcus Aureus, Micrococcus Luteus, Proteus Mirabilis, Pseudomonas Aeruginosa, Serratia Marcescens, Staphylococcus Aureus - MSRA, Staphylococcus Epidermidis, Staphylococcus Haemolyticus, Staphylococcus Hominis, Staphylococcus Saprophyticus, Trichophyton Mentagrophytes, Vancomycin Resistant Enterococcus Faecium, Vibrio Vulnificus, Salmonella, MRSA, Avian influenza virus (AIV), H7N1, Sars CoV-2 (Covid 19), Sars CoV-1, Mers, Noroviruses.

Preferably the disinfectant for carrying out the method is externally provided as a liquid. However, it is also possible that the HOCI is provided as a concentrate, as granules or in the form of tablets and that the method comprises the step of diluting or dissolving said concentrate, granules or tablets in water, preferably tap water.

Furthermore, an apparatus for disinfecting air, in particular for dispersing disinfectants, for use in a method as described above is provided, comprising a housing with an outlet opening and a nozzle, wherein the apparatus is configured to eject and/or disperse droplets, in particular an aerosol, of a disinfectant, wherein the apparatus comprises a pump, wherein the apparatus further comprises a micro sieve, wherein the micro sieve has openings, wherein a diameter of the openings is less than 10 µm.

The apparatus can be used in the method as described above. Any features or functions described above in connection with the inventive method can be applied to the apparatus accordingly and vice versa, where applicable.

Preferably the micro sieve is arranged essentially at the outlet opening.

By arranging the micro sieve essentially at the outlet opening, the disinfectant pumped through the nozzle by the pump can be pushed onto the micro sieve, in particular at a high velocity, so that the disinfectant is dispersed into a very fine aerosol by the micro sieve, where a mean droplet size of the produced droplets is preferably between 0.5 µm to 50 µm, further preferably between 1 µm to 10 µm, still further preferably between 2 µm to 8 µm and in particular preferably between 3 µm and 7 µm.

The micro sieve provides a high control of range of droplet size from within +/- 1µm or higher ranges.

The apparatus is preferably configured such that a disinfectant pumped through and ejected from the nozzle subsequently passes through the micro sieve to form droplets, in particular an aerosol.

Because of the micro sieve a disinfectant pushed through the micro sieve and dispersed from the apparatus as an aerosol will have a specific size distribution, which allows for the water content of the disinfectant to rapidly evaporate. Thus, the water content of the disinfectant functions as a carrier for, for example, hypochlorous acid molecules, which in turn function as a dense grid that creates a protective atmosphere.

Preferably the diameter of the openings is less than 5 µm, further preferably less than 3 µm, still further preferably less than 1 µm.

Preferably the pump is an air pump, in particular a Venturi pump, employing Bernoulli's principle to pump a disinfectant through the nozzle.

By using an air pump, the disinfectant can be pumped through the nozzle at high velocities, which prevents clogging of the nozzle.

In contrast to the known prior art ultrasonic dispersers for disinfectants, in the apparatus a disinfectant may be syphoned, preferably from a disinfectant reservoir, and/or pumped through the nozzle by using an air pump employing Bernoulli's principle. The use of an air pump allows for a precise control of pressure, droplet sizes and dispersion rates of the disinfectant.

While prior art dry or cold fogging machines are only capable of producing a dense fog or mist, the apparatus produces an aerosol of the disinfectant and disperses the disinfectant at dispersion rates which allow for a continuous disinfection for up to 24 hours a day with or without people present in the volume and/or area to be disinfected.

Preferably, the apparatus is configured to disperse a disinfectant at a dispersion rate between 0.5 ml/h and 100 ml/h. The dispersion rate may be controllable to be continuous or periodic or aperiodic. The dispersion rate may be an average dispersion rate. In combination with a disinfectant comprising electrolyzed and/or sterilized water containing hypochlorous acid, an unperceivable ultrafine fog or aerosol of disinfectant can be dispersed, which is breathable and can be inhaled by humans or animals without any harm to the humans or animals.

Preferably, the apparatus is configured to disperse a disinfectant at a dispersion rate between 1.0 ml/h and 50.0 ml/h, further preferably between 3.0 ml/h and 30.0 ml/h, still further preferably between 5.0 ml/h and 20.0 ml/h.

It is also conceivable that the apparatus is configured to disperse a disinfectant at a dispersion rate between 0.75 ml/h and 7.5 ml/h, further preferably between 1.0 ml/h and 3.0 ml/h, still further preferably between 1.5 ml/h and 2.0 ml/h.

The apparatus may comprise a moving or movable nozzle. With a moving or movable nozzle the distribution of the disinfectant in the volume can be improved.

The apparatus may comprise more than one air pump and/or more than one nozzle. For example, to disperse the disinfectant into a large volume and/or area, such as a cinema, a, preferably, high power air pump can be used. However, a high-power air pump may cause an increased noise level. Therefore, it can be advantageous that the apparatus comprises more than one of the smaller air pumps. Preferably the apparatus comprises at least two air pumps and/or nozzles, further preferably the apparatus comprises at least four air pumps and/or nozzles, still further preferably the apparatus comprises at least six air pumps and/or nozzles. When more than one air pump is used, the number of air pumps operated simultaneously may vary depending on the disinfection requirements.

The pressure on the air pump may be variable to control dispersion levels and particle size of the disinfectant.

Preferably the housing comprises a cavity, wherein the micro sieve is disposed in the cavity and wherein a mouth of the nozzle opens into the cavity.

A disinfectant pumped through the nozzle by the air pump is ejected from the mouth of the nozzle into the cavity. On the opposing side of the nozzle, the cavity opens to the outside through the outlet opening of the housing. The micro sieve is arranged preferably in the cavity, but close to the outlet opening so that a disinfectant ejected from the nozzle is pushed against the micro sieve, dispersed into a fine aerosol and ejected from the apparatus through the outlet opening.

The mouth of the nozzle may also be called an orifice.

Still further preferably, the mouth of the nozzle is configured to eject the disinfectant into a spray cone with an opening angle of at least 140°, preferably at least 150°, further preferably at least 160°.

For example, the mouth of the nozzle may have a conical shape with an opening angle of at least 140°, preferably at least 150°, further preferably at least 160°.

The apparatus may comprise an impeller to disperse the disinfectant. With an impeller-based dispersion exact quantities of a ultra-fine HOCI particles can be produced for small volumes, therefore providing the ability to sanitize air and create a disinfected breathable environment in volumes up to 20m³.

Still further preferably, the housing comprises a return passage, which fluidically connects to the cavity, wherein the return passage is configured to return disinfectant pooling in the cavity and/or not being ejected from the apparatus back to a disinfectant reservoir.

Disinfectant not being ejected from the apparatus may pool in the cavity between the nozzle and the micro sieve or the outlet opening. By providing a return passage, any unused disinfectant can be returned to a disinfectant reservoir. In addition, not atomized disinfectant is returned to the disinfectant reservoir. This prevents clogging of the apparatus.

The disinfectant reservoir may be part of the apparatus.

Still further preferably the nozzle comprises a rotation nozzle piece, wherein the rotation nozzle piece is configured to be rotated by operation of the air pump.

In particular, the rotation nozzle piece may comprise the mouth of the nozzle. By operation of the air pump the rotating nozzle piece pump is rotated. Disinfectant pumped though the nozzle is dispersed out of the mouth of the nozzle and accelerated in the radial direction by centrifugal forces from the rotation nozzle piece.

With the rotating nozzle piece to disperse disinfectant, the particle size of the disinfectant is reduced by the rotation of the rotating nozzle piece. The rotating nozzle piece may be called a Particle Rotation Accelerator.

The mouth, also called orifice, of the nozzle, which is preferably disposed in the rotation nozzle piece, preferably has an opening diameter of less than 3.0 mm, further preferably of less than 1.0 mm, particularly preferably of less than 0.5 mm, still further preferably of less than 0.3 mm, further preferably of less than 0.25 mm, particularly preferably of less than 0.1 mm.

The apparatus may furthermore comprise a charging unit to add electrical charge to the dispersed particles of the disinfectant. This allows for a better adhesion to certain air-borne pathogenic particles, which may carry electrostatic charges. The added charge can be positive or negative.

Furthermore preferably, the housing comprises a connecting section for connecting the apparatus to a disinfectant reservoir.

The connecting section can be connected to a valve of the disinfectant reservoir and by operation of the pump, preferably the air pump, a disinfectant is pumped out of the disinfectant reservoir through the nozzle and pushed against the micro sieve so that an aerosol of fine droplets of the disinfectant is ejected from the outlet opening of the apparatus.

Still further preferably the apparatus further comprises at least one sensor, wherein preferably the at least one sensor comprises a pump pressure sensor, a humidity sensor, a temperature sensor, an atmospheric pressure sensor, a dispersion rate sensor, a flow rate sensor, a reservoir level sensor.

With data from the at least one sensor, the dispersion rate can be adjusted to the specific application of use of the apparatus.

The apparatus may comprise or be combined with a HEPA-filter. Such a combination delivers ultrafine particles of HOCI combined with HEPA filtration to keep the air and surfaces inside a car or other small environments disinfected and safe.

The apparatus may have wireless connectivity. In particular, the apparatus may send and/or receive data, in particular from the sensors or the control device via BLE, 2G, 4G, 5G, Wi-Fi, LAN or NB-loT. In addition, the apparatus may be able to send and/or receive data from and to the internet. The apparatus may be connected to an external server, for example to a cloud computer. The external server may be a SaaS platform. The apparatus may be accessed, monitored, and controlled using mobile applications.

Preferably, the disinfectant reservoir of the apparatus comprises a connector for a water tap and a micro dosing system, in particular a micro dosing multi-channel system. Tap water can be filled into the disinfectant reservoir through the connector and the disinfectant can be produced by diluting or dissolving HOCI in the form of concentrates, granules or in the form of tablets in the tap water inside the disinfectant reservoir. The HOCI concentrate, granules or tablets are delivered into the water by the micro dosing system.

The micro dosing system can comprise, preferably multiple, peristaltic pumps to transfer the exact quantity of the concentrate, granules or tablets to the disinfectant reservoir.

Furthermore, the concentration of HOCI in the disinfectant stored in the disinfectant reservoir can be monitored using appropriate sensors. The sensors may be sensors to measure the concentration of HOCI in the disinfectant and/or in the air.

The disinfectant reservoir may also comprise an automatic stirring device to always ensure even concentration of HOCI in the disinfectant at all times.

The disinfectant reservoir may be a swap-in container. The disinfectant reservoir may be replaced when empty. The empty disinfectant reservoir may be refilled by a specialized provider. The disinfectant reservoir may also comprise a pressure sensor. By measuring the back pressure in the disinfectant reservoir, the remaining disinfectant in the disinfectant reservoir can be measured.

This system, i.e. the disinfectant reservoir or the apparatus comprising the disinfectant reservoir, can be a closed loop system made from hospital grade Stainless Steel and coated with anti-microbial ZnO film to protect against any build-up of germs or pathogens on the exposed surfaces.

Furthermore, the disinfectant reservoir may be configured light-tight. A light-tight disinfectant reservoir may be advantageous because the disinfectant may be reactive to sunlight.

Still further a disinfectant for a method for disinfecting air as described above is provided, wherein the disinfectant comprises electrolyzed and/or sterilized water containing hypochlorous acid, wherein the disinfectant further comprises at least one additive, wherein the at least one additive preferably comprises an essential oil, in particular cinnamon oil, carvacrol oil, thymol oil, cajaput oil, clove oil, eucalyptus oil, sage oil or tea tree oil.

In addition, a filtration and/or sanitation device for filtration and/or sanitization of air is proposed, comprising at least a first layer comprising silver ion nanoparticles and a second layer comprising a TiO₂ coated grid and an assembly of UV lamps.

The filtration and/or sanitation device can be used in the method and/or with the apparatus as described above. Any features or functions described above in connection with the inventive method or apparatus can be applied to the filtration and/or sanitation device accordingly and vice versa, where applicable.

The aim of the filtration and/or sanitation device is to remove all pathogens in the air at any point in time. The filtration and/or sanitation device may use a range of sensors to calibrate each aspect of the filtration and/or sanitation device and its functions to clean, disinfect and sanitize the air up to 99.99999%.

The TiO₂ coated grid of the second layer is preferably photocatalytic and produces oxygen when activated by UV light. Thus, the UV lamps are preferably configured to emit, in particular high-power, UV-light onto the TiO₂ coated grid.

Preferably the assembly comprises at least two, further preferably at least four, still further preferably at least eight, UV lamps. The UV lamps preferably emit UV light at approx. 254 nm.

In a preferred embodiment the TiO₂ coated grid is configured as a mesh encapsulating the UV lamps. With this configuration safety is enhanced.

The combination of the first and the second layer provides high ORP potential for sanitizing any unwanted pathogens in the air.

Further preferably the filtration and/or sanitation device comprises at least one, further preferably at least two, layers of honeycomb carbon (110mm) (activated/catalytic). The combination of two layers of honeycomb carbon removes bacteria, odors, VOCs and HCHO from the air up to F4 level.

The filtration and/or sanitation device may further comprise a negative ion generator to produce negative ions. The produced negative ions may be used for improving the air quality.

Still further the filtration device may comprise a multi-layered cold plasma reactor with, preferably four, cold plasma tubes. The cold plasma tubes may be encapsulated in TiO₂ coated Meshes to generate positive and negative ions as well as low volume ozone to ensure that the air around is protected.

Also, the filtration and/or sanitation device may comprise a HEPA 110mm Filter according to E13 - H13 standard. The HEPA 110mm Filter may be a medical grade filter. The HEPA 110mm Filter ensures that all particles are filtered and no residue is there in the air to give an average PM 2.5 count of less than 30 as well as reduction in PM 1.0.

The filtration and/or sanitation device may be integrated with the apparatus for disinfecting air as described above.

The filtration and/or sanitation device may have wireless connectivity. The filtration and/or sanitation device may send and/or receive data, in particular from the sensors or the control device via BLE, 2G, 4G, 5G, Wi-Fi, LAN or NB-loT. In addition, the filtration and/or sanitation device may be able to send or receive data from and to the internet. The filtration and/or sanitation may be connected to an external server, for example to a cloud computer. The external server may be a SaaS platform. The filtration and/or sanitation device be accessed, monitored, and controlled using mobile applications.

The filtration and/or sanitation device can be connected to an ERV/HRV unit to bring in fresh air with energy recovery. All ERV/HRV units independently filter and sanitize air before bringing it in. The filtration and/or sanitation device can be connected to a fresh air unit. A fresh air unit functions similarly to the ERV/HRV but without energy recovery. Also the filtration and/or sanitation device can be connected to a negative pressure high volume return air extraction unit for removing air as expelled in the room. This system can be used in conjunction with the ERV/HRV or fresh air system.

All these units can be controlled using wall-based controls and can also communicate by using BLE, 2G, 4G, 5G, Wi-Fi, LAN or NB-loT. All data can be accessed, monitored and controlled using Apps as well as a SaaS platform.

A further solution to the problem solved by the invention is the provision of a system for disinfecting air, configured for carrying out a method as described above, wherein the system comprises at least one sensor and a control device, wherein the control device is configured to receive measurement data from the sensor, wherein the control device is configured to, preferably dynamically, determine a required concentration of hypochlorous acid in a volume to be disinfected and/or a dispersion rate of a disinfectant based on the measurement data.

The control device, for example a microprocessor or a computer or the like, receives measurement data from the sensors. The sensors can be part of an apparatus as previously described or they can be arranged in a volume and/or area to be disinfected.

The control device receives data from the sensors and determines and/or adjusts the concentration of hypochlorous acid in the volume and/or the dispersion rate of the disinfectant into the volume to be disinfected.

Preferably, the system comprises a heating, ventilation and air conditioning system (HVAC) configured to distribute and/or disperse the disinfectant.

It is furthermore preferred that the system comprises at least one, preferably more than one, apparatus for disinfecting air as described above and/or at least one filtration and/or sanitation device as described above.

A disinfectant reservoir, comprising a container is also provided, wherein the container has an interior coating, preferably of ZnO, and/or wherein the container has an exterior coating, preferably of ZnO, further preferably a lamination of a ZnO film.

Still further preferably the container of the disinfectant reservoir is a double-walled container.

The container can be made from hospital grade stainless steel.

Furthermore, the disinfectant reservoir may be configured light-tight.

The disinfectant reservoir may be part of or combined with an apparatus as described above.

Yet another solution to the problem is the provision of a computer program product comprising instructions which, when the program is executed by a computer, cause the computer to carry out a determination of a concentration of hypochlorous acid in a volume to be disinfected and/or of a dispersion rate of a disinfectant, according to a method as described above based on the measurement data of at least one sensor.

The measurement data may comprise at least one of the parameters: volume to be disinfected, a concentration of hypochlorous acid in the volume and/or in the disinfectant, and/or an air exchange rate, and/or a ventilation status, and/or humidity, and/or atmospheric pressure, and/or surface area and/or material of surfaces present in the volume, and/or characteristics of walls surrounding the volume, for example material and surface structure, and/or a decay rate of disinfectant, and/or number of people present or expected in the volume, and/or temperature and/or pH of the air in the volume, and/or concentration of gases in the volume, for example the concentration of CO₂, Cl₂, ClO₂, HOCl, N₂, H₂CO, H₂O, ClO, HCl, and/or at least one of PM 0.3, PM 1.0, PM 2.5, PM 10, HCHO concentration, TVOC concentration.

The computer program product may comprise a mobile application, which can be carried out on a portable device such as a smartphone, a smartwatch, a tablet and so forth.

Preferably, a user may use the mobile application to adjust the parameters of a system for disinfecting air as described above. For example, the user may adjust the dispersion rate, or he may adjust parameters such as the room humidity or he may specify surfaces present in the volume and/or area.

### Brief description of the figures

The invention is described in the following with reference to the accompanying figures.
- Fig. 1: shows a system for disinfecting air,
- Fig. 2a: shows a cut out view of an apparatus for dispersing a disinfectant,
- Fig. 2b: shows an exploded view of an apparatus for dispersing a disinfectant,
- Fig. 2c: shows a cross sectional view of an apparatus for dispersing a disinfectant,
- Fig. 3: shows a nozzle for an apparatus for dispersing a disinfectant,
- Fig. 4: shows an apparatus for dispersing a disinfectant mounted on a disinfectant reservoir,
- Fig. 5: shows a schematic of a system for disinfecting air, Fig. 6 shows filtration and/or sanitation device, and
- Fig. 7: shows a schematic view of a filtration and/or sanitation device.

### Detailed description of the figures

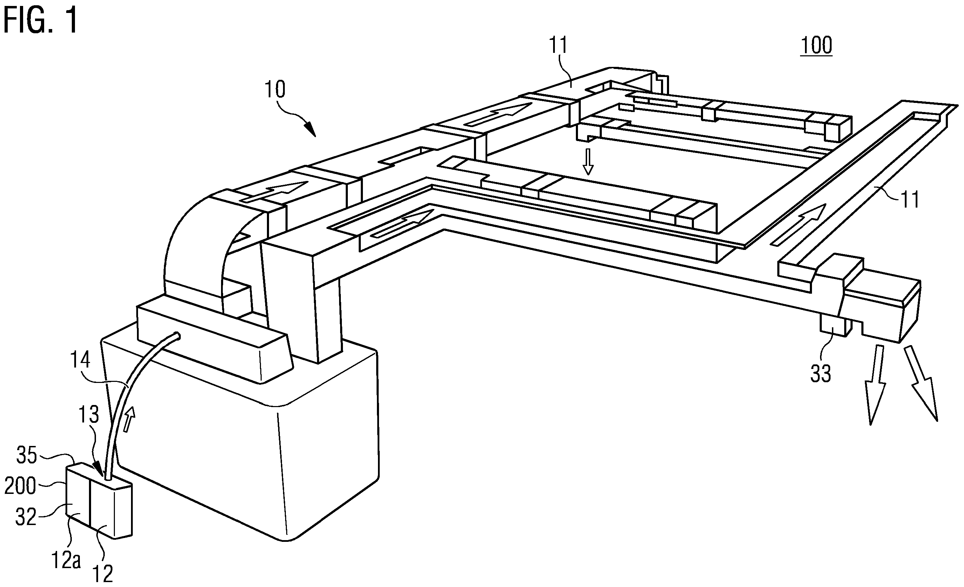

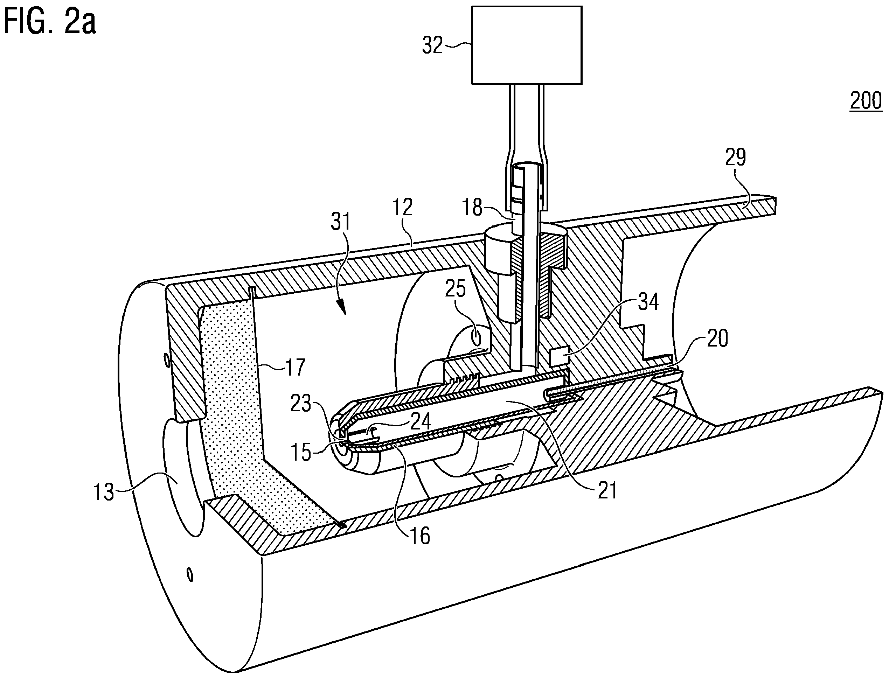

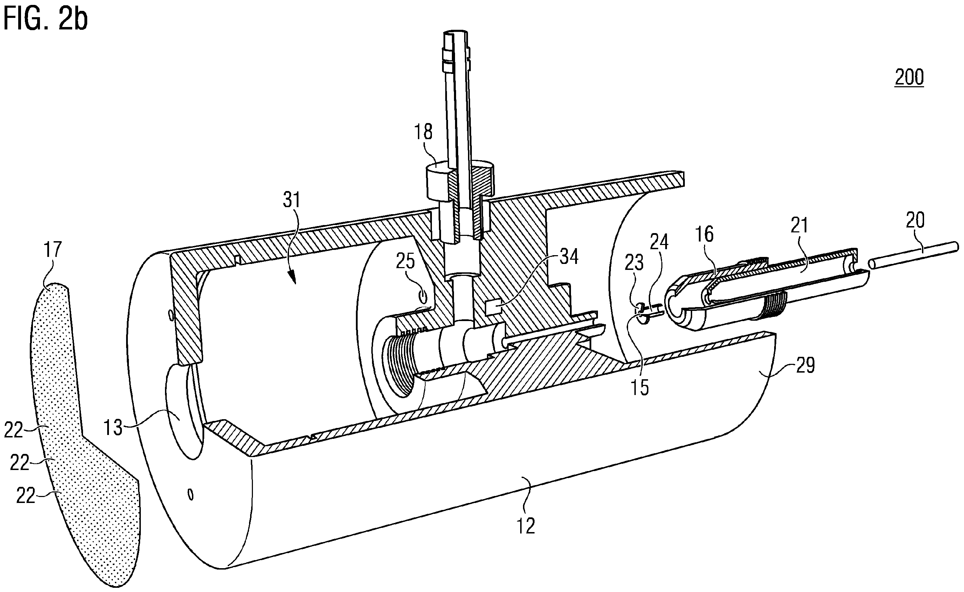

Fig. 1 shows a system 100 for disinfecting air. The system 100 comprises an apparatus 200 for dispersing a disinfectant as well as an HVAC system 10 comprising ducts 11. The apparatus 200 has a shell 12a. Inside the shell 12a a housing 12 comprising an outlet opening 13, as shown in Figs. 2a and 2b, is disposed. Furthermore, an air pump 32 is arranged inside shell 12a. An aerosol of a disinfectant is ejected through the outlet opening 13 of the apparatus 200 and guided to the HVAC system 100 via a delivery tube 14. The disinfectant comprises electrolyzed and/or sterilized water containing hypochlorous acid. During operation of the system 100, the aerosol of the disinfectant is distributed via the ducts 11 into a volume to be disinfected, for example into the rooms of a building. The system further comprises a sensor 33 for measuring, for example the concentration of hypochlorous acid in the air, an air exchange rate, atmospheric pressure or room humidity. A control device 35 is disposed inside shell 12a. Based on measurement data of the sensor 33, and of additional sensors 34 of the apparatus (Figs. 2a and 2b), the control device adjusts a dispersion rate of the disinfectant such, that the concentration of the hypochlorous acid in the air in the rooms of the building is continuously between 0.05 mg/m³ and 2.0 mg/m³.

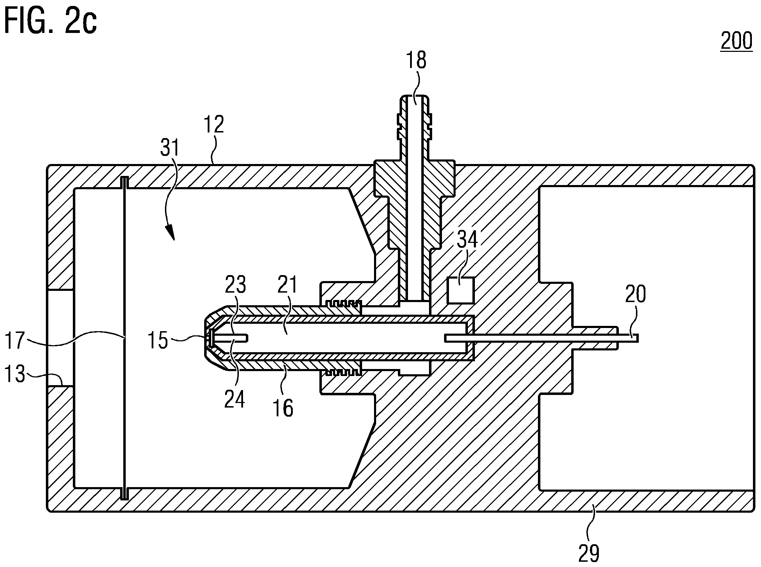

Fig. 2a shows a cut out view of an apparatus 200 for dispersing a disinfectant, which can be used in the system 100 of Fig. 1. Fig. 2b shows an exploded view of the apparatus of Fig. 2a. Fig. 2c shows a cross sectional view of the apparatus of Figs. 2a and 2b.

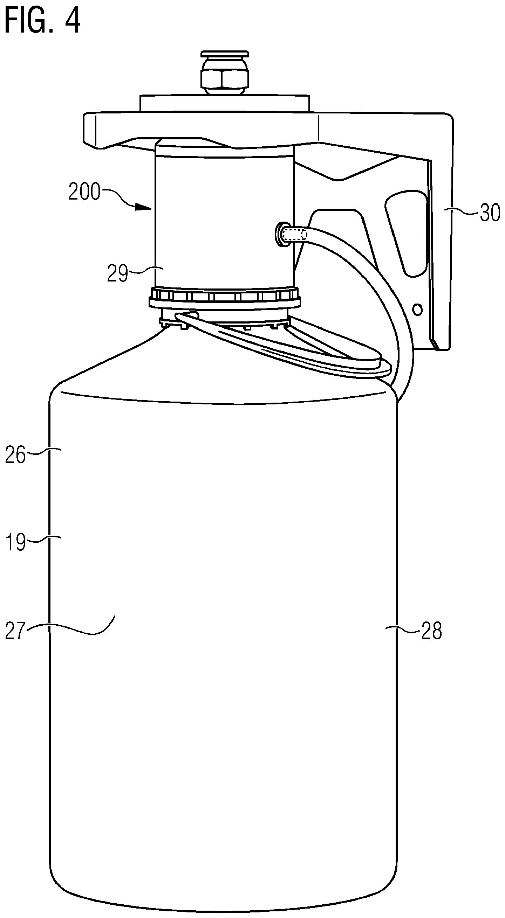

The apparatus 200 comprises a housing 12 having an outlet opening 13. A cavity 14 is disposed inside the housing 12. A mouth 15 of a nozzle 16 opens into the cavity 31. A micro sieve 17 is arranged essentially at the outlet opening 13. The housing 12 further comprises a connector 18 for an air pump 32. When the air pump 32 is operated, a low-pressure region is created inside the nozzle 16 and a disinfectant is syphoned from a disinfectant reservoir 19 (Fig. 4) through an inlet pipe 20. The disinfectant is mixed with air in a mixing chamber 21 of the nozzle 16 and ejected from the mouth 15 of the nozzle 16 into the cavity 31. Because of the high ejection velocity of the disinfectant from the nozzle 16, the disinfectant is pushed against the micro sieve 17. The micro sieve 17 has openings 22 with a diameter of about 1 µm to 10 µm. Disinfectant passing the micro sieve 17 is dispersed into an aerosol with droplet sizes in the range of preferably 3 µm to 7 µm. The aerosol is then ejected through the outlet opening 13 either directly into a volume to be disinfected or the aerosol is guided via a delivery tube 14 into the ducts 11 of an HVAC system 100 (Fig. 1). The nozzle 16 further comprises a rotation nozzle piece 23 configured as an atomization device 24. The rotation nozzle piece 23 comprises the mouth 15 of the nozzle 16. The rotation nozzle piece 23 is mounted rotatably in the nozzle 16 and is rotated by the operation of the air pump 32. Because of the rotation of the rotation nozzle piece 23 the disinfectant ejected from mouth 15 of nozzle 16 is accelerated in the radial direction to form a spray cone with an opening angle of approximately 160°. The apparatus 200 also comprises a sensor 34, configured for measuring a flow rate of the disinfectant through the nozzle or to measure the air pump pressure.

The housing 12 further comprises a return passage 25 fluidically connected to the cavity 31 and the disinfectant reservoir 19 (Fig. 4). Any disinfectant pooling inside cavity 31 is returned to the disinfectant reservoir 19 via return passage 25.

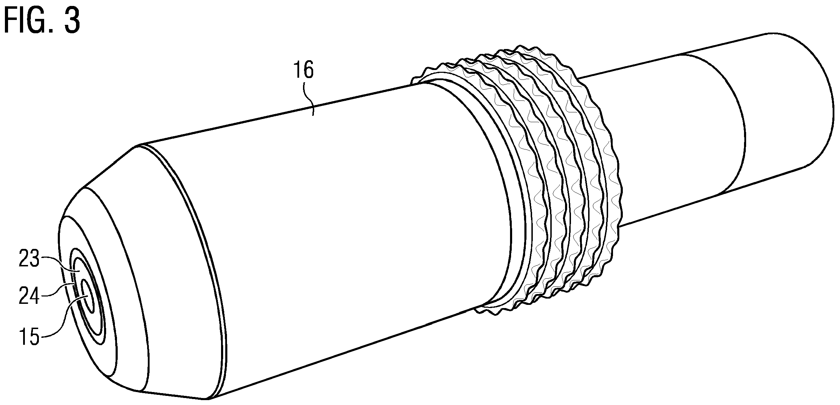

Fig. 3 shows a perspective view of the nozzle 16 of the apparatus 200. The nozzle 16 comprises a rotation nozzle piece 23 configured as an atomization device 24. The mouth 15 of the nozzle 15 is disposed in the rotation nozzle piece 23. A disinfectant syphoned from a disinfectant reservoir 19 and pumped through nozzle 16 is ejected from mouth 15 of the rotation nozzle piece 23 into a spray cone with an opening angle of about 160°.

Fig. 4 shows the apparatus 200 mounted on top of a disinfectant reservoir 19. The disinfecting reservoir 19 is configured as a container 26 having an exterior coating 27 of a ZnO laminate. On the inside the container 26 is also covered with ZnO. The container 26 is configured as a double-walled container 28. The apparatus 200 is connected to the disinfectant reservoir 19 via a connecting section 29 (Figs. 2a and 2b). The disinfectant reservoir 19 also comprises a latch 30 for mounting the disinfectant reservoir to a wall.

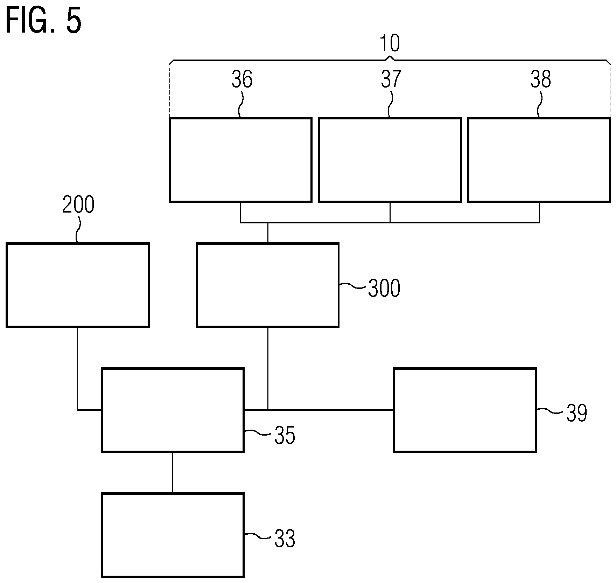

Fig. 5 shows a schematic view of a variant of the system 100 for disinfecting air of Fig. 1. The system 100 comprises an HVAC system 10, which an ERV (energy recovery ventilation) or HRV (heat recovery ventilation) unit 36, a fresh air unit 37 and a return air extractor 38. The system 100 further comprises an apparatus 200 for dispersing a disinfectant. The apparatus 200 disperses an aerosol of the disinfectant with droplet sizes in the range of preferably 3 µm to 7 µm. The disinfectant comprises electrolyzed and/or sterilized water containing hypochlorous acid. The aerosol of the disinfectant is distributed either directly into a volume to be disinfected, e.g. into the rooms of a building, or via the HVAC system 10. The system 100 furthermore comprises a set of sensors 33, which are configured to measure at least one of the parameters: volume to be disinfected, a concentration of hypochlorous acid in the volume and/or in the disinfectant, and/or an air exchange rate, and/or a ventilation status, and/or humidity, and/or atmospheric pressure, and/or surface area and/or material of surfaces present in the volume, and/or characteristics of walls surrounding the volume, for example material and surface structure, and/or a decay rate of disinfectant, and/or number of people present in the volume, and/or temperature and/or pH of the air in the volume, and/or concentration of gases in the volume, for example the concentration of CO₂, Cl₂, ClO₂, HOCl, N₂, H₂CO, H₂O, ClO, HCl, and/or at least one of PM 0.3, PM 1.0, PM 2.5, PM 10, HCHO concentration, TVOC concentration. The sensor data from the sensors 33 is transmitted to a control device 35, which based on the sensor data determines the required concentration of hypochlorous acid in the volume, i.e. in the rooms of the building and the actual concentration of hypochlorous acid in the volume. The control device 35 then adjusts the dispersion rate of the apparatus 200 for the disinfectant to control the concentration of hypochlorous acid in the volume to be continuously between 0.05 mg/m³ and 2.0 mg/m³, preferably between 0.1 mg/m³ and 1.0 mg/m³. Since the aerosol of the disinfectant has droplet sizes in the range of preferably 3 µm to 7 µm, the droplets evaporate rapidly resulting a disinfectant in the form of sub-micron size aerosol particles, which never settle down because of gravity.

The system 100 further comprises a filtration and/or sanitation device 300. The filtration and/or sanitation device 300 may be coupled to the HVAC system 10 to filter and/or sanitize air distributed by the HVAC system 10. If the disinfectant is not distributed by the apparatus 200 directly into a volume to be disinfected, the disinfectant may pass through the filtration and/or sanitation device 300 in order to disinfect said filtration and/or sanitation device 300.

The system 100 may also be connected to an external server 39, for example to a cloud computer, which runs a SaaS platform. The external server 39 may be configured to control and monitor the system 100. In addition, the external server 39 may provide information on the system and its components to an operator of the system or to the mobile devices of persons in the building to allow them to evaluate the safety and disinfection level of the building.

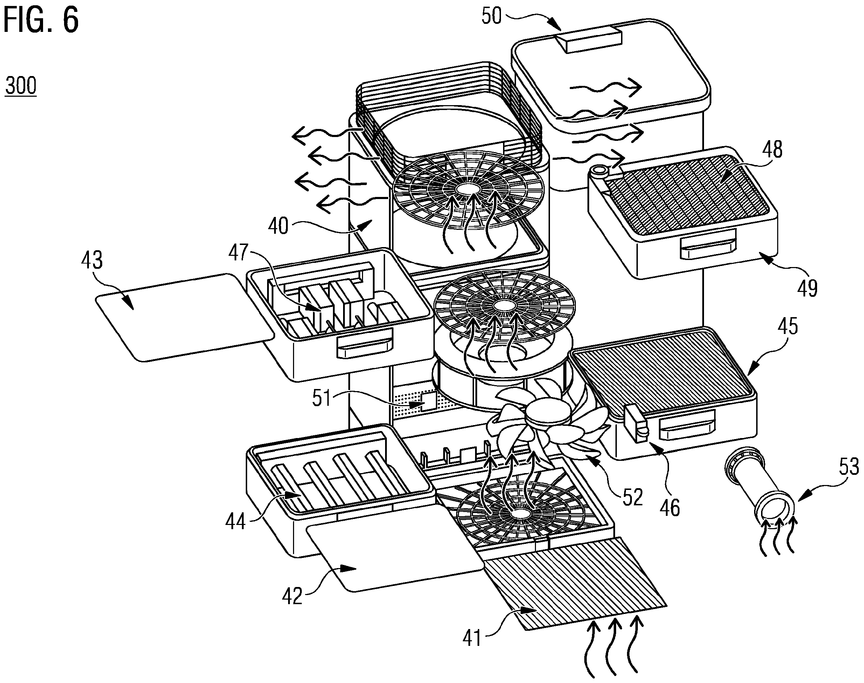

Fig. 6 shows an exploded view of a filtration and/or sanitation device 300 which can be used in the system 100 of Fig. 5. The filtration and/or sanitation device comprises a housing 40 comprising a multi-layer filter arrangement. The multi-layer filter arrangement comprises roughly from bottom to top a filter 41 coated with silver ion nanoparticles, and a first TiO₂ coated grid 42 and a second TiO₂ coated grid 43 and an assembly of 254 nm UV lamps 44. The TiO₂ coated grids 42, 43 are photocatalytic and produce oxygen when activated by UV light from the UV lamps 44. In addition, the filtration and/or sanitation device 300 comprises two layers of honeycomb carbon 45 (activated/catalytic), a negative ion generator 46 to produce negative ions, and a multi-layered low ozone cold plasma reactor 47. Near the top, the filtration and/or sanitation device 300 may comprise a medical grade HEPA Filter 48 to ensures that all particles are filtered and no residue is in the air to give an average PM 2.5 count of less than 30 as well as reduction in PM 1.0. In the embodiment shown in Fig. 6 the filtration and/or sanitation device 300 further comprises a HOCI Dispersion and Tank unit 49. For control of the filtration and/or sanitation device 300 a touch display 50 is provided. A multi Parameter IAQ sensor 51 is used to monitor the filtration and/or sanitation device 300. Finally, a high volume fan 52 and a fresh air inlet 53 are part of the filtration and/or sanitation device 300. The order of the components of the filtration and/or sanitation device 300 may be changed depending on design choices.

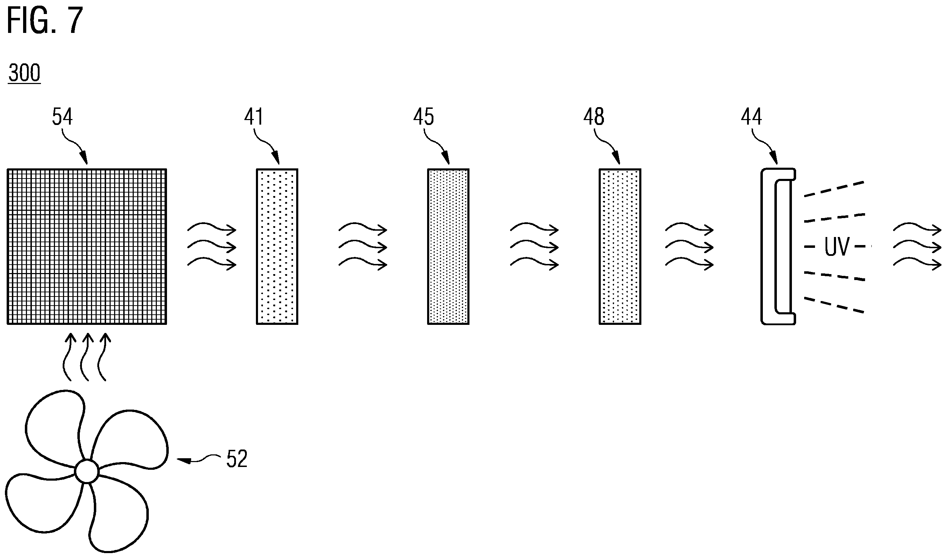

Fig. 7 shows a schematic view of a filtration and/or sanitation device 300. Air to be filtered is pushed through an energy recovery module 54 by the high volume fan 52. The air then passes a coarse filter 41 coated with silver ion nanoparticles and two layers of honeycomb carbon 45. After passing a HEPA Filter 48, the air is sanitized by UV light from UV lamps 44. In contrast to the filtration and/or sanitation device 300 of Fig. 6, in the embodiment of Fig. 7 the UV lamps 44 are the final stage of the filtration and/or sanitation device 300. Furthermore, the energy recovery module 54 may be replaced with an electronic non return damper (not shown).

### List of reference numerals

- 100: System for disinfecting air
- 200: Apparatus for dispersing a disinfectant
- 300: Filtration and/or sanitation device
- 10: HVAC system
- 11: Duct
- 12: Housing
- 12a: Shell
- 13: Outlet opening
- 14: Delivery tube
- 15: Mouth
- 16: Nozzle
- 17: Micro sieve
- 18: Connector
- 19: Disinfectant reservoir
- 20: Inlet pipe
- 21: Mixing chamber
- 22: Opening
- 23: Rotation nozzle piece
- 24: Atomization device
- 25: Return passage
- 26: Container
- 27: Exterior coating
- 28: Double-walled container
- 29: Connecting section
- 30: Latch
- 31: Cavity
- 32: Air pump
- 33: Sensor
- 34: Sensor
- 35: Control device
- 36: ERV/HRV unit
- 37: Fresh air unit
- 38: Return air extractor
- 39: External server
- 40: Housing
- 41: Filter coated with silver ion nanoparticles
- 42: First TiO₂ coated grid
- 43: Second TiO₂ coated grid
- 44: UV lamps
- 45: Honeycomb carbon
- 46: Negative ion generator
- 47: Cold plasma reactor
- 48: HEPA Filter
- 49: HOCI Dispersion and Tank unit
- 50: Touch display
- 51: IAQ sensor
- 52: High volume fan
- 53: Fresh air inlet
- 54: Energy recovery module

## Claims

1. Method for disinfecting air, wherein a disinfectant is dispersed into a volume to be disinfected, wherein the disinfectant comprises electrolyzed and/or sterilized water containing hypochlorous acid, **characterized in that** a dispersion rate of the disinfectant is adjusted such, that a concentration of the hypochlorous acid in the volume is continuously between 0.05 mg/m³ and 2.0 mg/m³.

2. Method according to claim 1, **characterized in that** the concentration is adjusted to be between 0.1 mg/m³ and 2.0 mg/m³, preferably between 0.1 mg/m³ and 1.0 mg/m³, further preferably between 0.2 mg/m³ and 0.5 mg/m³.

3. Method according to claim 1 or 2, **characterized in that** the disinfectant is dispersed as an aerosol, wherein a mean value of a droplet size distribution is between 0.5 µm and 50 µm, preferably between 1 µm and 10 µm, further preferably between 2 µm and 8 µm, particularly preferably between 3 µm and 7 µm, and/or that the disinfectant has a pH between 4 and 7, preferably between 5 and 6, and/or that the hypochlorous acid has a concentration in the disinfectant between 10 ppm and 1.000 ppm, preferably between 100 ppm and 500 ppm, further preferably between 200 ppm and 400 ppm, and/or that the disinfectant has a redox potential between 500 mV and 1.500 mV, preferably between 700 mV and 1.200 mV, further preferably between 900 mV and 1.000 mV.

4. Method according to any one of the preceding claims, **characterized in that** the disinfectant comprises at least one additive, wherein the additive preferably is present in the disinfectant with a weight percentage or a volume percentage between 0.01 % and 5.0 %, further preferably between 0.25 % and 1.0 %, still further preferably between 0.5 % and 0.75 %, and/or wherein preferably the at least one additive comprises an essential oil, wherein the essential oil further preferably is at least one out of cinnamon oil, carvacrol oil, thymol oil, cajuput oil, clove oil, eucalyptus oil, sage oil, tea tree oil.

5. Method according to any one of the preceding claims, **characterized in that** the disinfectant in the volume is present in the form of a sub-micron size particles.

6. Method according to any one of the preceding claims, **characterized in that** the concentration and/or the dispersion rate is adjusted based on at least one of the parameters: volume to be disinfected, concentration of hypochlorous acid in the volume and/or in the disinfectant, and/or air exchange rate, and/or ventilation status, and/or humidity, and/or atmospheric pressure, and/or surface area and/or material of surfaces present in the volume, and/or characteristics of walls surrounding the volume, for example material and surface structure, and/or a decay rate of disinfectant, and/or number of people present in the volume, and/or temperature and/or pH of the air in the volume, and/or concentration of gases in the volume, for example the concentration of CO₂, Cl₂, ClO₂, HOCl, N₂, H₂CO, H₂O, ClO, HCl, and/or at least one of PM 0.3, PM 1.0, PM 2.5, PM 10, HCHO concentration, TVOC concentration.

7. Method according to any one of the preceding claims, **characterized in that** the air of the volume is filtered and/or sanitized, wherein preferably the air is filtered and/or sanitized using silver ion nanoparticles, and wherein further preferably the air is filtered and/or sanitized using a UV light activated TiO₂ coated grid producing oxygen.

8. System (100) for disinfecting air, configured for conducting a method according to any one of claims 1 to 7, **characterized in that** the system comprises at least one sensor (33, 34) and a control device (35), wherein the control device (35) is configured to receive measurement data from the at least one sensor (33, 34), wherein the control device (35) is configured to, preferably dynamically, determine a required concentration of hypochlorous acid in a volume to be disinfected and/or a dispersion rate of a disinfectant based on the measurement data.

9. A computer program product comprising instructions which, when the program is executed by the control device (35) of the system according to claim 8, cause the control device (35) to carry out the determination of the concentration of hypochlorous acid in the volume to be disinfected and/or of the dispersion rate of the disinfectant, based on the measurement data of the at least one sensor, according to the method of claim 6.

## Patentansprüche

1. Verfahren zur Desinfektion von Luft, bei dem ein Desinfektionsmittel in ein zu desinfizierendes Volumen dispergiert wird, wobei das Desinfektionsmittel elektrolysiertes und/oder sterilisiertes Wasser umfasst, das hypochlorige Säure enthält, **dadurch gekennzeichnet, dass** eine Dispersionsrate des Desinfektionsmittels so eingestellt wird, dass eine Konzentration der hypochlorigen Säure in dem Volumen kontinuierlich zwischen 0,05 mg/m³ und 2,0 mg/m³ liegt.

2. Verfahren gemäß Anspruch 1, **dadurch gekennzeichnet, dass** die Konzentration so eingestellt wird, dass sie zwischen 0,1 mg/m³ und 2,0 mg/m³, bevorzugt zwischen 0,1 mg/m³ und 1,0 mg/m³, weiter bevorzugt zwischen 0,2 mg/m³ und 0,5 mg/m³, liegt.

3. Verfahren gemäß Anspruch 1 oder 2, **dadurch gekennzeichnet, dass** das Desinfektionsmittel als Aerosol dispergiert wird, wobei ein Mittelwert einer Tröpfchengrößenverteilung zwischen 0,5 µm und 50 µm, bevorzugt zwischen 1 µm und 10 µm, weiter bevorzugt zwischen 2 µm und 8 µm, besonders bevorzugt zwischen 3 µm und 7 µm, liegt, und/oder dass das Desinfektionsmittel einen pH-Wert zwischen 4 und 7, bevorzugt zwischen 5 und 6, aufweist, und/oder dass die hypochlorige Säure eine Konzentration im Desinfektionsmittel zwischen 10 ppm und 1. 000 ppm, bevorzugt zwischen 100 ppm und 500 ppm, weiter bevorzugt zwischen 200 ppm und 400 ppm, aufweist, und/oder dass das Desinfektionsmittel ein Redoxpotential zwischen 500 mV und 1.500 mV, bevorzugt zwischen 700 mV und 1.200 mV, weiter bevorzugt zwischen 900 mV und 1.000 mV, aufweist.

4. Verfahren gemäß einem der vorhergehenden Ansprüche, **dadurch gekennzeichnet, dass** das Desinfektionsmittel mindestens ein Additiv umfasst, wobei das Additiv bevorzugt mit einem Gewichts- oder Volumenprozentsatz zwischen 0,01 % und 5,0 %, weiter bevorzugt zwischen 0,25 % und 1. 0 %, noch weiter bevorzugt zwischen 0,5 % und 0,75 %, vorliegt, und/oder wobei das mindestens eine Additiv bevorzugt ein ätherisches Öl umfasst, wobei das ätherische Öl weiter bevorzugt mindestens eines aus Zimtöl, Carvacrolöl, Thymolöl, Cajuputöl, Nelkenöl, Eukalyptusöl, Salbeiöl, Teebaumöl ist.

5. Verfahren gemäß einem der vorhergehenden Ansprüche, **dadurch gekennzeichnet, dass** das Desinfektionsmittel in dem Volumen in Form von Partikeln mit einer Größe im Submikronbereich vorliegt.

6. Verfahren gemäß einem der vorhergehenden Ansprüche, **dadurch gekennzeichnet, dass** die Konzentration und/oder die Dispersionsrate basierend auf mindestens einem der folgenden Parameter eingestellt ist: zu desinfizierendes Volumen, Konzentration der hypochlorigen Säure im Volumen und/oder im Desinfektionsmittel, und/oder Luftwechselrate, und/oder Belüftungszustand, und/oder Luftfeuchtigkeit, und/oder Luftdruck, und/oder Oberfläche und/oder Material der im Volumen vorhandenen Oberflächen, und/oder Eigenschaften der das Volumen umgebenden Wände, z.B. Material und Oberflächenstruktur, und/oder eine Abklingrate des Desinfektionsmittels, und/oder die Anzahl der im Volumen anwesenden Personen, und/oder Temperatur und/oder pH-Wert der Luft im Volumen, und/oder Konzentration von Gasen im Volumen, z.B. die Konzentration von CO₂, Cl₂, ClO₂, HOCl, N₂, H₂CO, H₂O, ClO, HCl, und/oder mindestens eines von PM 0.3, PM 1.0, PM 2.5, PM 10, HCHO-Konzentration, TVOC-Konzentration.

7. Verfahren gemäß einem der vorhergehenden Ansprüche, **dadurch gekennzeichnet, dass** die Luft des Volumens gefiltert und/oder desinfiziert wird, wobei die Luft bevorzugt unter Verwendung von Silberionen-Nanopartikeln gefiltert und/oder desinfiziert wird, und wobei die Luft weiter bevorzugt unter Verwendung eines UV-Licht-aktivierten TiO₂-beschichteten Gitters, das Sauerstoff erzeugt, gefiltert und/oder desinfiziert wird.

8. System (100) zum Desinfizieren von Luft, das zur Durchführung eines Verfahrens gemäß einem der Ansprüche 1 bis 7 ausgebildet ist, **dadurch gekennzeichnet, dass** das System mindestens einen Sensor (33, 34) und eine Steuervorrichtung (35) umfasst, wobei die Steuervorrichtung (35) so konfiguriert ist, dass sie Messdaten von dem mindestens einen Sensor (33, 34) empfängt, wobei die Steuervorrichtung (35) so konfiguriert ist, dass sie, bevorzugt dynamisch, eine erforderliche Konzentration an hypochloriger Säure in einem zu desinfizierenden Volumen und/oder eine Dispersionsrate eines Desinfektionsmittels basierend auf den Messdaten bestimmt.

9. Computerprogrammprodukt umfassend Anweisungen, die, wenn das Programm durch die Steuervorrichtung (35) des Systems gemäß Anspruch 8 ausgeführt wird, die Steuervorrichtung (35) veranlassen, die Bestimmung der Konzentration an hypochloriger Säure in dem zu desinfizierenden Volumen und/oder die Dispersionsrate des Desinfektionsmittels basierend auf den Messdaten des mindestens einen Sensors gemäß dem Verfahren des Anspruchs 6 durchzuführen.

## Revendications

1. Procédé pour désinfecter l'air, dans lequel un désinfectant est dispersé dans un volume à désinfecter, le désinfectant comprenant de l'eau électrolysée et/ou stérilisée contenant de l'acide hypochloreux, **caractérisé en ce qu'**un taux de dispersion du désinfectant est ajusté de telle manière qu'une concentration de l'acide hypochloreux dans le volume est en continu comprise entre 0,05 mg/m³ et 2,0 mg/m³.

2. Procédé selon la revendication 1, **caractérisé en ce que** la concentration est ajustée pour être comprise entre 0,1 mg/m³ et 2,0 mg/m³, de préférence entre 0,1 mg/m³ et 1,0 mg/m³, encore de préférence entre 0,2 mg/m³ et 0,5 mg/m³.

3. Procédé selon la revendication 1 ou 2, **caractérisé en ce que** le désinfectant est dispersé sous forme d'aérosol, une valeur moyenne d'une distribution de taille de gouttelette étant comprise entre 0,5 um et 50 um, de préférence entre 1 um et 10 um, encore de préférence entre 2 um et 8 um, particulièrement de préférence entre 3 um et 7 pm, et/ou que le désinfectant a un pH compris entre 4 et 7, de préférence entre 5 et 6, et/ou que l'acide hypochloreux a une concentration dans le désinfectant compris entre 10 ppm et 1 000 ppm, de préférence entre 100 pm et 500 ppm, encore de préférence entre 200 ppm et 400 ppm, et/ou que le désinfectant a un potentiel redox compris entre 500 mV et 1 500 mV, de préférence entre 700 mV et 1 200 mV, encore de préférence entre 900 mV et 1 000 mV.

4. Procédé selon l'une quelconque des revendications précédentes, **caractérisé en ce que** le désinfectant comprend au moins un additif, l'additif étant de préférence présent dans le désinfectant avec un pourcentage en poids ou un pourcentage en volume compris entre 0,01 % et 5,0 %, encore de préférence entre 0,25 % et 1,0 %, encore encore de préférence entre 0,5 % et 0,75 % et/ou de préférence l'au moins un additif comprend une huile essentielle, l'huile essentielle étant encore de préférence au moins une de l'huile de cannelle, de l'huile de carvacrol, de l'huile de thymol, de l'huile de cajeput, de l'huile de clou de girofle, de l'huile d'eucalyptus, de l'huile de sauge, de l'huile d'arbre à thé.

5. Procédé selon l'une quelconque de revendications précédente, **caractérisé en ce que** le désinfectant dans le volume est présent sous forme de particules de taille sub-micrométrique.

6. Procédé selon l'une quelconque des revendications précédentes, **caractérisé en ce que** la concentration et/ou le taux de dispersion est ajusté sur la base d'au moins un des paramètres : volume à désinfecter, concentration de l'acide hypochloreux dans le volume et/ou le désinfectant, et/ou vitesse d'échange d'air, et/ou statut de ventilation, et/ou humidité, et/ou pression atmosphérique, et/ou surface et/ou matériau de surfaces présent dans le volume, et/ou caractéristiques des parois entourant le volume, par exemple matériau et structure de surface, et/ou une vitesse de déclin de désinfectant, et/ou nombre de personnes présentes dans le volume, et/ou température et ou pH de l'air dans le volume, et/ou concentration de gaz dans le volume, par exemple la concentration de CO₂, Cl₂, ClO₂, HOCl, N₂, H₂CO, H₂O, ClO, HCl et/ou au moins un de PM 0,3, PM 1,0, PM 2,5, PM 10, concentration de HCHO, concentration de TVOC.

7. Procédé selon l'une quelconque des revendications précédentes, **caractérisé en ce que** l'air du volume est filtré ou aseptisé, l'air étant de préférence filtré ou aseptisé en utilisant des nanoparticules d'ion d'argent et en outre de préférence l'air étant filtré ou aseptisé en utilisant une grille revêtue de TiO₂ activé par la lumière UV produisant de l'oxygène.

8. Système (100) pour désinfecter l'air, configuré pour effectuer un procédé selon l'une quelconque des revendications 1 à 7, **caractérisé en ce que** le système comprend au moins un capteur (33, 34) et un dispositif de contrôle (35), le dispositif de contrôle (35) étant configuré pour recevoir des données de mesure de l'au moins un capteur (33, 34), le dispositif de contrôle (35) étant configuré pour, de préférence dynamiquement, déterminer une concentration requise d'acide hypochloreux dans un volume à désinfecter et/ou un taux de dispersion d'un désinfectant sur la base des données de mesure.

9. Produit de programme informatique comprenant des instructions qui, quand le programme est exécuté par le dispositif de contrôle (35) du système selon la revendication 8, entraîne le dispositif de contrôle (35) à réaliser la détermination de la concentration de l'acide hypochloreux dans le volume à désinfecter et/ou du taux de dispersion du désinfectant, sur la base des données de mesure de l'au moins un capteur, selon le procédé de la revendication 6.
